# EUROPEAN PATENT SPECIFICATION

(11) **EP 3 009 152 B1**
(45) Date of publication and mention of the grant of the patent: **11.07.2018**
(21) Application number: 15187762.8
(22) Date of filing: 30.09.2015
(51) Int. Cl.: A61L 2/07, A61L 2/20, B01B 1/00

(54) **METHOD FOR STERILIZING A CONTAINER**
STERILISIERUNGSVERFAHREN ZUM STERILISIEREN EINES BEHÄLTERS
PROCÉDÉ DE STÉRILISATION D'UN RÉCIPIENT

(30) Priority: 16.10.2014 IT PR20140071
(43) Date of publication of application: 20.04.2016
(73) Proprietor: Gea Procomac S.p.A., 43038 Sala Baganza (PR) (IT)
(72) Inventor: ABELLI, Paolo, 43123 Parma (IT); ANTIGA, Simone, 43013 Languirano (PR) (IT)
(74) Representative: Dondi, Silvia

(56) References cited:
- WO-A1-02/078753
- WO-A2-98/34649
- WO-A2-2008/011181
- DE-U1-202010 011 419

## Description

The present invention relates to a method for sterilizing a container.

The reference sector is the bottling of so-called "sensitive" food products, i.e. products that are particularly sensitive to bacteriological contamination and oxidation, such as, for example, isotonic drinks, juices, nectars, soft drinks, tea, milk-based drinks, coffee-based drinks, etc., for which it is fundamental to prevent any microbiological contamination throughout the packaging stages.

Within this context, the focus is on the decontamination of the primary packaging, whether it is a bottle, a pouch, or a cardboard container; the decontamination is performed before the packaging is filled.

Decontamination techniques of the prior art for containers and the relative closing systems (spouts, caps, etc.) envisage the use of chemical agents (e.g. aqueous solutions containing peracetic acid or hydrogen peroxide), or the application of radiation or the use of heat.

For example, a first chemical sterilisation method for sterilizing the inside walls of a container envisages the following steps:
- vaporizing an aqueous solution containing hydrogen peroxide into the container (e.g. bottle);
- making the hydrogen peroxide vapours condense on the inside walls of the container (bringing the walls themselves to a temperature less than or equal to the condensation temperature of the peroxide vapours);
- making the condensed agent evaporate using hot air so as to "activate" it;
- drying the inside walls of the container.

A second chemical sterilisation method envisages keeping the hydrogen peroxide constantly in the vapour state. In that case, the peroxide does not need "to be activated".

However, in the second method the temperature of the inside walls of the container must always be higher than the condensation temperature of the peroxide vapours so as to prevent their condensation: this condition can be technically and economically laborious to implement.

For example, document US2010/0202919 proposes a vaporizer for sterilizing plastic containers comprising a cylindrical housing having a heating plate as its base. An aqueous solution containing hydrogen peroxide is injected into the vaporizer through a longitudinal central conduit at the end of which a nozzle is assembled, which nozzle sprays the solution onto the heating plate so as to make it evaporate. A gaseous means (e.g. air) is introduced into the cylinder and, through a conveyor, sent towards the heating plate. This gaseous means acts as a carrier for the vaporized sterilizing agent, i.e. it carries it towards the outside of the cylinder, hence towards the containers to be sterilized.

The main limit of the solutions using the first method and air as a carrier is connected with the need to deposit the hydrogen peroxide condensate onto all the surfaces to be treated, which are not always easy to reach.

Let's consider, for example, containers with a complex geometry (e.g. undercuts or handles) for which condensation only takes place in high treatment times, which require oversized sterilizing apparatus.

The use of water vapour as a carrier (instead of air) makes the condensation of the hydrogen peroxide on the surfaces to be treated easier, but this solution poses another problem.

In fact, it is not possible to be sure of the complete evaporation of the liquid hydrogen peroxide injected into the vapour flow. This is due to undesired condensation of the vapour itself, which contrast the completion of the evaporation of the hydrogen peroxide.

On the other hand, it is actually the complete evaporation of the peroxide that allows the concentration of the sterilizing agent which condenses on the surfaces to be monitored, ensuring that pre-established sterility values are obtained and remain constant over time.

The use of water vapour as a carrier is described in US7708959, in which a predefined dose of aqueous solution containing liquid hydrogen peroxide is injected into a vapour flow. This mixture is conveyed towards a sterilization chamber in which the spout or cap of a container to be sterilized is placed. In this solution, the vapour performs the following functions:
- it causes the evaporation of the sterilizing agent (and therefore the activation of the peroxide);
- it acts as a carrier, i.e. it carries the sterilizing agent.

However, to keep the concentration of the sterilizing agent under control and to ensure the complete evaporation of the peroxide, the structural complexity must be increased.

For example, document US7708959 envisages a shifting device for the injector so as to move it away from the vapour when the injection of the peroxide is not required. Alternatively, a cooling device is provided in proximity to the injector so as to contrast the increase in the peroxide temperature before injection into the vapour flow.

In this context, the technical task underpinning the present invention is to provide a method for sterilizing a container, which obviate the drawbacks of the prior art cited above.

In particular, an object of the present invention is to provide a method for sterilizing a container that ensure the desired sterility degree is obtained and maintained over time, without having to increase the structural complexity.

The set technical task and the specified objects are substantially attained by a method for sterilizing a container, comprising the technical characteristics as set out in one or more of the accompanying claims.

Further characteristics and advantages of the present invention will more fully emerge from the indicative, and therefore non-limiting, description of a preferred but not exclusive embodiment of a method for sterilizing a container, as illustrated in the accompanying drawings, in which:
- figure 1 schematically illustrates a first embodiment of a sterilizing apparatus for sterilizing a container, according to the claimed method;
- figure 2 schematically illustrates a second embodiment of a sterilizing apparatus for sterilizing a container, according to the claimed method.

With reference to the figures, 1 is used to indicate a sterilizing apparatus for sterilizing a container 100. In particular, the container 100 is a bottle made of high-density polyethylene (known by the acronym HDPE).

The sterilizing apparatus 1 comprises a mixing chamber 2 having a first opening 3 of admission of water vapour.

For example, the water vapour comes from the supply services of the site where the apparatus 1 operates. Alternatively, the water vapour is generated by a dedicated generating unit 9.

Preferably, the water vapour is at a pressure comprised between about 2 bar and 3 bar and, therefore, at a temperature comprised between 120°C and 145°C.

The sterilizing apparatus 1 comprises means for introducing 4 an aqueous compound containing a liquid sterilizing agent inside the mixing chamber 2. In particular, the liquid sterilizing agent is hydrogen peroxide.

Preferably, the concentration of hydrogen peroxide in the aqueous compound is about 35%.

Preferably, the means for introducing 4 the aqueous compound comprise a spray nozzle 5. For example, the spray nozzle 5 is a compressed air or piezoelectric spray nozzle.

In an alternative not illustrated, the means for introducing 4 consist of a conduit that has the aqueous compound drip into the mixing chamber 2.

In an alternative not illustrated, the means for introducing 4 consist of an ejector that draws the aqueous compound into the vapour flow contained in the mixing chamber 2.

Inside the mixing chamber 2, coming into contact with the water vapour (at the temperature and pressure indicated above), the aqueous compound containing the sterilizing agent also starts to evaporate.

Hence a mixture of water vapour and evaporated sterilizing agent is generated which is discharged from the mixing chamber 2 through a second opening 6.

Originally, before being delivered to the container 100 to be sterilized, the mixture thus obtained is heated by a heating unit 7 which promotes the complete evaporation of the sterilizing agent and keeps the mixture in the gaseous state.

Such heating unit 7 is placed upstream of a treatment chamber 8 of the container 100 to be sterilized.

In the embodiments described and illustrated herein, the treatment chamber 8 is the internal volume of the bottle 100, i.e. the space delimited by the walls and the bottom of the bottle 100.

In an alternative embodiment (not illustrated), the treatment chamber 8 is a chamber containing the bottle 100, therefore it is the external walls of the bottle 100 that are treated.

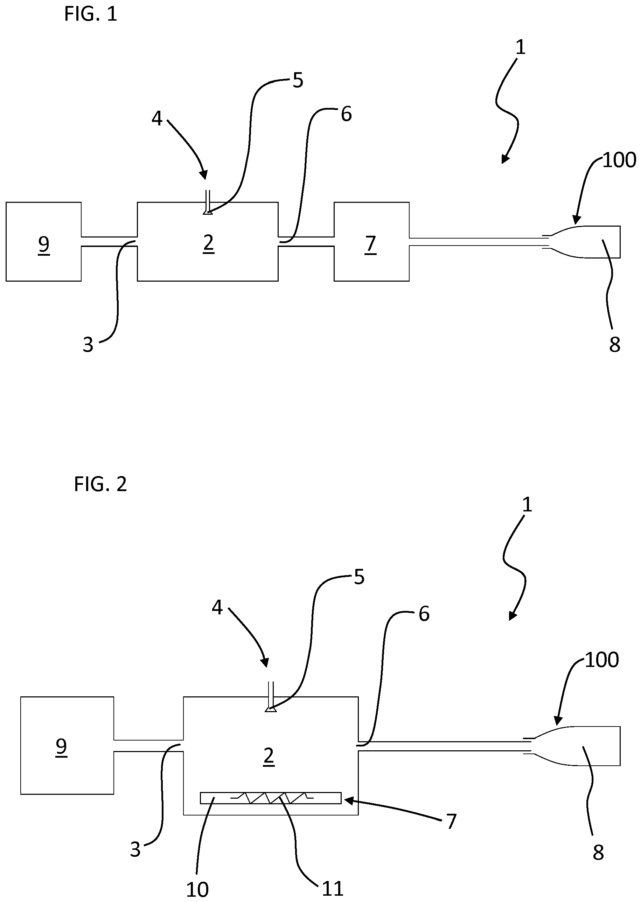

In a first embodiment, illustrated in fig. 1, the heating unit 7 is placed downstream of the mixing chamber 2 so as to receive the mixture of water vapour and partially evaporated sterilizing agent from the second opening 6 of the mixing chamber 2.

For example, the heating unit 7 consists of an electric heat exchanger or a vapour heat exchanger of the known type.

In a second embodiment, illustrated in fig. 2, the heating unit 7 is located inside the mixing chamber 2.

For example, the heating unit 7 consists of a heat exchanger comprising a metal plate 10 inside which at least one electric resistance 11 is arranged.

Alternatively, the heating unit 7 consists of a vapour heat exchanger comprising a metal plate appropriately shaped to make the vapour circulate.

The method for sterilizing a container, according to the present invention, is described below.

The water vapour, for example coming from the dedicated generating unit 9 or from the supply services of the site, is entered into the mixing chamber 2 and the aqueous compound containing the liquid sterilizing agent is introduced into the vapour flow. This introduction preferably takes place by injection, for example, by spraying the aqueous compound through the spray nozzle 5.

As already mentioned above, the high temperature of the water vapour makes the aqueous compound containing the sterilizing agent partially evaporate too. Thus, the mixture of water vapour and partially evaporated sterilizing agent is obtained.

In the first embodiment, the mixture thus obtained passes into the heating unit 7 placed downstream of the mixing chamber 2.

Advantageously, the heating unit 7 allows the evaporation of the aqueous compound (hence of the sterilizing agent) to be completed and the mixture to be kept in the gaseous state.

In particular, the heating unit 7 is sized so as to be able to evaporate a maximum quantity of liquid pre-established according to the required degree of sterilization.

After identifying the working point, i.e. the operating temperature, of the heating unit 7 which allows the liquid component of the mixture (coming from the mixing chamber 2) to evaporate completely, this temperature condition is maintained over time thanks to a feedback control.

In the second embodiment, the mixture is heated directly inside the mixing chamber 2, which contains the heating unit 7.

For example, the mixture impacts against the heated metal plate 10 which completes its evaporation and guarantees the maintenance of the gaseous state.

Subsequently, the mixture is drawn into the container 100, brushing against its inside walls.

In particular, the inside walls of the container 100 are kept at a temperature less than or equal to the condensation temperature of the hydrogen peroxide vapour, i.e. less than 60°C.

In this way, the hydrogen peroxide condenses on the walls. Subsequently, it is evaporated by means of the delivery of hot air.

The concentration of sterilizing agent in the mixture is determined by measuring the flow rate of vapour and the flow rate of sterilizing agent at the inlet to the mixing chamber 2, a concentration which is kept constant through a feedback control that operates on the dedicated valves so as to keep the flow rates within a pre-established range.

From the above description, the characteristics of the method for sterilizing a container, according to the present invention, are clear, as are the advantages.

In particular, the method proposed herein use pressurized water vapour as a carrier, which makes it easier to treat medium sized-large bottles and with complex geometry, such as the bottles used in the dairy industry.

By heating the gaseous mixture (formed by water vapour and hydrogen peroxide) prior to delivering it to the container, the complete evaporation of the sterilizing agent is guaranteed, which allows the concentration of the condensed sterilizing agent to be kept under control (preventing its condensation prior to the contact with the walls of the container), thus ensuring that the desired degree of sterility is obtained and maintained over time.

To do so, it is sufficient to insert a heat exchanger just downstream of the mixing chamber or directly inside it without having to increase the structural complexity of the apparatus.

## Claims

1. Method for sterilizing a container (100), comprising the steps of:
generating a flow of water vapour;
introducing an aqueous compound containing a liquid sterilizing agent into the flow of water vapour so as to obtain a mixture of water vapour and partially evaporated sterilizing agent;
delivering said mixture to the container (100) to be sterilized,
**characterized in that** it comprises the steps of:
heating the mixture of water vapour and partially evaporated sterilizing agent prior to delivering it to said container (100);
maintaining constant the concentration of the sterilizing agent in said mixture by a feedback control;
the concentration of sterilizing agent in the mixture being determined by measuring the flow rate of vapour and the flow rate of sterilizing agent at the inlet to a mixing chamber (2), wherein the concentration is kept constant through the feedback control that operates on dedicated valves so as to keep the flow rates within a pre-established range.

2. Method according to claim 1, wherein the step of introducing the aqueous compound containing the liquid sterilizing agent into the flow of water vapour takes place within a mixing chamber (2) and the step of heating the mixture of water vapour and partially evaporated sterilizing agent prior to delivering it to said container (100) is carried out in a heating unit (7) that is located downstream said mixing chamber (2).

3. Method according to claim 1, wherein the step of introducing the aqueous compound containing the liquid sterilizing agent into the flow of water vapour takes place within a mixing chamber (2) and the step of heating the mixture of water vapour and partially evaporated sterilizing agent prior to delivering it to said container (100) is carried out in a heating unit (7) that is placed inside said mixing chamber (2).

4. Method according to any of the previous claims, wherein the step of introducing the aqueous compound containing the liquid sterilizing agent into the flow of water vapour takes place by injection.

## Patentansprüche

1. Sterilisierungsverfahren zum Sterilisieren eines Behälters (100), umfassend die folgenden Schritte: Erzeugen eines Wasserdampfstroms;
Einführen einer wässrigen Verbindung, enthaltend ein flüssiges Sterilisierungsmittel, in den Wasserdampfstrom, sodass eine Mischung aus Wasserdampf und teilweise verdampftem Sterilisierungsmittel erhalten wird;
Zuführen der Mischung zum zu sterilisierenden Behälter (100),
**dadurch gekennzeichnet, daß** es die folgenden Schritte umfasst:
Erhitzen der Mischung aus Wasserdampf und teilweise verdampftem Sterilisierungsmittel vor deren Zuführung zum Behälter (100);
konstantes Aufrechterhalten der Konzentration des Sterilisierungsmittels in der Mischung durch eine Feedback-Kontrolle,
wobei die Konzentration des Sterilisierungsmittels in der Mischung durch das Messen der Durchflussmenge des Dampfs und der Durchflussmenge des Sterilisierungsmittels am Einlass zu einer Mischkammer (2) ermittelt wird, wobei die Konzentration durch die Feedback-Kontrolle konstant aufrechterhalten wird, die auf entsprechende Ventile einwirkt, sodass die Durchflussmengen innerhalb eines vorgegebenen Wertebereichs beibehalten werden.

2. Verfahren nach Anspruch 1, wobei der Schritt zum Einführen der wässrigen Verbindung, enthaltend das flüssige Sterilisierungsmittel, in den Wasserdampfstrom in einer Mischkammer (2) stattfindet und der Schritt zum Erhitzen der Mischung aus Wasserdampf und teilweise verdampftem Sterilisierungsmittel vor deren Zuführung zum Behälter (100) in einer Heizeinheit (7) durchgeführt wird, die sich stromabwärts der Mischkammer (2) befindet.

3. Verfahren nach Anspruch 1, wobei der Schritt zum Einführen der wässrigen Verbindung, enthaltend das flüssige Sterilisierungsmittel, in den Wasserdampfstrom in einer Mischkammer (2) stattfindet und der Schritt zum Erhitzen der Mischung aus Wasserdampf und teilweise verdampftem Sterilisierungsmittel vor deren Zuführung zum Behälter (100) in einer Heizeinheit (7) durchgeführt wird, die sich in der Mischkammer (2) befindet.

4. Verfahren nach einem der vorhergehenden Ansprüche, wobei der Schritt zum Einführen der wässrigen Verbindung, enthaltend das flüssige Sterilisierungsmittel, in den Wasserdampfstrom durch Einspritzung stattfindet.

## Revendications

1. Procédé de stérilisation d'un récipient (100), comprenant les étapes de :
générer un flux de vapeur d'eau ;
introduire un composant aqueux contenant un agent de stérilisation liquide dans le flux de vapeur d'eau de sorte à obtenir un mélange de vapeur d'eau et un agent de stérilisation partiellement évaporé ;
envoyer ledit mélange au récipient (100) à stériliser,
**caractérisé en ce qu'**il comprend les étapes de :
chauffer le mélange de vapeur d'eau et d'agent de stérilisation partiellement évaporé avant de l'envoyer au dit récipient (100) ;
maintenir constante la concentration de l'agent de stérilisation dans ledit mélange par un contrôle par rétroaction ,
la concentration d'agent de stérilisation dans le mélange étant déterminée en mesurant le débit de vapeur et le débit d'agent de stérilisation à l'entrée d'une chambre de mélange (2), dans lequel la concentration est maintenue constante à travers le contrôle par rétroaction qui opère sur des vannes dédiées de sorte à maintenir les débits dans une fourchette préétablie.

2. Procédé selon la revendication 1, dans lequel l'étape consistant à introduire le composant aqueux contenant l'agent de stérilisation liquide dans le flux de vapeur d'eau se déroule à l'intérieur d'une chambre de mélange (2) et l'étape consistant à chauffer le mélange de vapeur d'eau et l'agent de stérilisation partiellement évaporé avant de l'envoyer au dit récipient (100) est effectuée dans une unité de chauffage (7) étant située en aval de ladite chambre de mélange (2).

3. Procédé selon la revendication 1, dans lequel l'étape consistant à introduire le composant aqueux contenant l'agent de stérilisation liquide dans le flux de vapeur d'eau se déroule à l'intérieur d'une chambre de mélange (2) et l'étape consistant à chauffer le mélange de vapeur d'eau et l'agent de stérilisation partiellement évaporé avant de l'envoyer au dit récipient (100) est effectuée dans une unité de chauffage (7) étant placée à l'intérieur de ladite chambre de mélange (2).

4. Procédé selon l'une quelconque des revendications précédentes, dans lequel l'étape consistant à introduire le composant aqueux contenant l'agent de stérilisation liquide dans le flux de vapeur d'eau se déroule par injection.
